## Europäisches Patentamt
## European Patent Office
## Office européen des brevets

(19)

(11) Veröffentlichungsnummer: **0 128 388**
**B1**

# EUROPÄISCHE PATENTSCHRIFT

(12)

(51) Int. Cl.⁴: **A 61 M 1/00**, A 61 M 27/00

(45) Veröffentlichungstag der Patentschrift:
10.06.88

(21) Anmeldenummer: 84105553.6

(22) Anmeldetag: 16.05.84

(54) Vorrichtung zum Absaugen von Sekretflüssigkeit aus einer Wunde.

(30) Priorität: 11.06.83 DE 3321151

(43) Veröffentlichungstag der Anmeldung:
19.12.84 Patentblatt 84/51

(45) Bekanntmachung des Hinweises auf die Patenterteilung:
10.06.88 Patentblatt 88/32

(84) Benannte Vertragsstaaten:
AT BE CH DE FR GB IT LI LU NL SE

(56) Entgegenhaltungen:
CH-A-499 328
DE-A-1 766 793
DE-A-1 932 540
DE-A-2 637 681
DE-B-2 207 887
GB-A-2 044 105

(73) Patentinhaber: Beck, Walter, Obere Häslibachstrasse 87, CH-8700 Küsnacht (CH)
Patentinhaber: Werner, Margrit, Philipp-Wasserburgstrasse 30, D-6500 Mainz-Gonsenheim (DE)

(72) Erfinder: Beck, Walter, Obere Häslibachstrasse 87, CH-8700 Küsnacht (CH)
Erfinder: Berger, Siegfried, Hauptstrasse 6, D-7314 Wernau (DE)
Erfinder: Werner, Heinz-Peter, Prof. Dr., Philipp-Wasserburgstrasse 30, D-6500 Mainz (DE)

(74) Vertreter: Patentanwälte Phys. Bartels Dipl.-Ing. Fink Dr.-Ing. Held, Lange Strasse 51, D-7000 Stuttgart 1 (DE)

EP 0 128 388 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung betrifft eine Vorrichtung zum Absaugen von Sekretflüssigkeit aus einer Wunde mit einem Drain, der über einen Schlauch mit einem Sekretsammelbehälter dicht verbunden ist, und einer Pumpe, die mit dem Sekretsammelbehälter, dem Schlauch und dem Drain zu einem sterilen System vereinigt ist.

Bei einer bekannten Vorrichtung dieser Art (DE-A-1 932 540) ist der Sekretsammelbehälter mittels einer Schraubkappe verschlossen, durch die hindurch nicht nur der zum Drain führende Schlauch eingeführt ist, sondern auch ein zweiter Schlauch, der zu einer Vakuumpumpe führt, die ebenso wie die anderen Teile der Vorrichtung steril in einem Beutel enthalten sind. Nach dem Gebrauch wird das gesamte System einschließlich der Pumpe weggeworfen, was die Kosten erhöht. Nachteilig ist ferner, daß die Saugleistung dieser Vorrichtung von der gleichbleibenden Saugleistung der Pumpe abhängt und deshalb in vielen Fällen nicht optimal ist. Ferner muß wegen der unlösbaren Verbindung des Drains mit dem Sekretsammelbehälter der Drain von der Körperoberfläche her in die Wunde eingeführt werden. Dabei besteht die Gefahr, daß der Drain mit Erregern, die sich auf der Hautoberfläche und in den obersten Hautschichten befinden, in Berührung kommt und diese Erreger in die Wunde verschleppt werden.

Kostengünstiger als diese bekannte Vorrichtung sind diejenigen sterilen Systeme, bei denen der Sekretsammelbehälter evakuiert ist. Aufgrund des Unterdruckes im Sekretsammelbehälter ist am Drain ständig eine Saugwirkung vorhanden, deren Intensität zu Beginn des Absaugvorganges ihr Maximum hat und mit zunehmender Füllung des Behälters geringer wird. In vielen Fällen muß aber bei diesen Systemen eine Hämatombildung in Kauf genommen werden. Zwar kann durch Massage ein Hämatomabbau erreicht werden, jedoch wird durch die Hämatombildung das Erreichen der Mobilisierungsphase des Patienten verzögert.

Bei einer anderen bekannten Vorrichtung zum Absaugen von Sekretflüssigkeit aus einer Wunde (CH-A-499 328) ist der Sekretsammelbehälter als ein sich selbsttätig rückstellender Faltenbalg ausgebildet, um gleichzeitig als Saugpumpe zu wirken. Auch hier ist deshalb ständig die volle Saugleistung wirksam. Damit der Drain nicht von der Körperoberfläche her durch das der Wunde benachbarte Gewebe eingeführt werden muß, ist das dem Drain abgekehrte Ende des Schlauches lösbar in eine Aufnahme des Sekretsammelbehälters eingesteckt. Es ist dadurch möglich, den Schlauch mittels einer speziellen Nadel von innen nach außen durch das Gewebe zu ziehen. Allerdings muß hierfür eine Trennstelle zwischen Schlauch und Sekretsammelbehälter in Kauf genommen werden, an der Erreger in das System und von diesem aus in die Wunde gelangen können.

Der Erfindung liegt die Aufgabe zugrunde, eine Vorrichtung zum Absaugen von Sekretflüssigkeit aus einer Wunde zu schaffen, die nicht nur vollkommen steril, sondern auch kostengünstig ist und besser als die bekannten Vorrichtungen in ihrer Saugleistung an die Erfordernisse angepaßt werden kann. Diese Aufgabe löst eine Vorrichtung mit den Merkmalen des Anspruches 1.

Durch den Einsatz einer Schlauchpumpe braucht das sterile, bakteriell geschlossene System nur aus dem Drain, dem Sekretsammelbehälter und dem ihn mit dem Drain verbindenden Schlauch zu bestehen. Die Pumpe braucht deshalb nicht weggeworfen zu werden, sondern kann immer wieder verwendet werden, und zwar ohne jedesmal steril gemacht werden zu müssen. Dies reduziert die Kosten. Ferner braucht der Sekretsammelbehälter nicht formstabil zu sein, wie dies bei den evakuierten Sekretsammelbehältern erforderlich ist, was die Kosten ebenfalls vermindert. Von besonderem Vorteil ist ferner, daß die Saugleistung der Vorrichtung sich selbsttätig an die Erfordernisse anpaßt, das heißt unabhängig vom Füllstand des Sekretsammelbehälters eine minimale Saugwirkung aufrechterhalten wird, solange keine Sekretflüssigkeit aus dem Drain abfließt, jedoch dann, wenn Sekretflüssigkeit fließt, die Saugwirkung auf den erforderlichen Wert erhöht wird. Die Schlauchpumpe verhindert deshalb auch einen Stillstand der Sekretflüssigkeit oder ein Zurückfließen, was ebenfalls wichtig ist. Weiterhin ist von Vorteil, daß der Sekretsammelbehälter ein relativ großes Volumen haben kann, so daß ein Wechsel überflüssig wird, wohingegen bei den bekannen Systemen in Abständen von sechzehn bis vierundzwanzig Stunden ein Wechsel bzw. ein Öffnen des Systems unerläßlich ist.

Hinzu kommt der weitere Vorteil, daß bei einer Ausbildung des Sammelbehälters als Plastikbeutel an dessen Aussehen zu erkennen ist, ob die Wunde dicht oder nicht dicht ist.

Der Sekretsensor ist vorzugsweise an dem zwischen der Schlauchpumpe und dem Drain liegenden Abschnitt des Schlauches angeordnet. Je geringer dabei der Abstand vom Drain ist, desto frühzeitiger kann der Sekretsensor das Anfallen von Sekretflüssigkeit oder das Ende einer Absonderung von Sekretflüssigkeit erkennen. Damit der Sekretsensor berührungsfrei arbeitet, was aus Hygienegründen wichtig ist, ist er bei einer bevorzugten Ausführungsform ein kapazitiver oder induktiver Geber, dessen Kapazität bzw. Induktivität davon abhängig ist, ob sich in der aus Kunststoff oder Gummi bestehenden Schlauchleitung Sekretflüssigkeit befindet oder nicht. Bei einem kapazitiven Sekretsensor können beispielsweise die beiden Elektroden den Schlauch schellenartig auf einem Teil seines Umfangs umfassen.

Da in der Sekretflüssigkeit Luftblasen enthalten sein können und der Sensor dann, wenn er eine solche Luftblase erkennt, die Schlauchpumpe auf ihre Mindestleistung umschalten würde, was in unerwünschter Weise zu einem ständigen Umschalten der Pumpe zwischen ihrer Mindestleistung und der erhöhten Leistung führen würde, ist bei einer bevorzugten Ausführungsform ein zweiter Sekretsensor am Schlauch in Strömungsrichtung gegenüber dem ersten Sekretsensor versetzt angeordnet, der beim Auftreten von im Sekret mitgeführter Luftblasen eine Umschaltung der Schlauchpumpe auf die minimale Leistung durch den ersten Sekretsensor verhindert. Vorzugsweise wird dies bei Verwendung gleich ausgebildeter Sekretsensoren dadurch erreicht, daß die zur Auslösung einer Umschaltung erforderlichen Signale der beiden Sensoren durch eine logische Schaltung miteinander verknüpft sind. Eine Umschaltung der Pumpe auf ihre Mindestleistung erfolgt dann nur, wenn beide Sekretsensoren keine Sekretflüssigkeit erkennen. Wählt man den Abstand der beiden Sekretsensoren voneinander größer als die Länge einer Luftblase, dann kann eine Luftblase nicht mehr zu einer Umschaltung der Pumpe auf ihre Mindestleistung führen.

Die von dem Sekretsensor oder den Sekretsensoren erzeugten Signale können in verschiedener Weise für die Umschaltung der Schlauchpumpe ausgewertet werden. Bei einer wegen ihrer Einfachheit und Zuverlässigkeit vorteilhaften Ausführungsform liegt jeder vorhandene kapazitive oder induktive Sekretsensor im Schwingkreis eines Oszillators, dessen Ausgangsspannung hinsichtlich ihrer Amplitude vom Sensorsignal abhängig ist. Man kann dann die Ausgangsspannung des Oszillators oder der Oszillatoren nach einer Gleichrichtung und Verstärkung zur Ansteuerung einer Leistungsstufe verwenden, welche die für den Betrieb der Pumpe erforderliche Energie liefert.

Bei einer bevorzugten Ausführungsform hat die Intervallsteuerung Einstellelemente für eine veränderbare Einstellung der Größe der minimalen und der maximalen Saugleistung der Schlauchpumpe. Es ist dann eine optimale Anpassung der Saugleistung an die Erfordernisse möglich. Bei diesen Einstellelementen kann es sich beispielsweise um Drehknöpfe handeln. Aber auch eine Folientastatur, mittels deren beispielsweise unterschiedlich hohe Drehzahlen der Schlauchpumpe vorgegeben werden können, kommt in Frage.

Bei einer bevorzugten Ausführungsform ist die Schlauchpumpe außen an einem die Intervallsteuerung enthaltenden Gehäuse angeordnet. In diesem Gehäuse kann auch der Sekretsensor oder können die Sekretsensoren untergebracht sein, wodurch freiliegende Verbindungsleitungen vom Sensor oder den Sensoren zu der Intervallsteuerung entfallen, wie sie notwendig wären, wenn der Sensor oder die Sensoren im Abstand von diesem Gehäuse am Schlauch angeordnet wären. Die Anordnung des Sensors oder der Sensoren im Gehäuse der Intervallsteuerung bietet auch den Vorteil, Störeinflüsse von den Sensoren besser fernhalten zu können, als wenn sie sich außerhalb des Gehäuses befinden würden.

Um dennoch denjenigen Schlauchabschnitt, den der Sensor oder die Sensoren überwachen, ohne Schwierigkeiten in die unmittelbare Nähe des Sensors oder der Sensoren bringen zu können, ist vorteilhafterweise ein in das Gehäuse der Intervallsteuerung einführbarer Einschub vorgesehen, der mit einer Aufnahme für den Schlauch versehen ist, so daß nur der Einschub zusammen mit dem Schlauch in das Gehäuse eingeführt zu werden braucht, um die Sensoren wirksam werden zu lassen. Dabei kann der Einschub nicht nur so ausgebildet sein, daß zunächst der Schlauch an ihm festgelegt werden muß. Vielmehr kann der Einschub auch Teil des Einwegsystems sein, das nach Gebrauch weggeworfen wird.

Vorzugsweise ist für die Festlegung des Schlauches in der Schlauchpumpe ebenfalls ein Halter vorgesehen. Bei diesem Halter, der zusammen mit dem an ihm festgelegten Schlauch in eine Aufnahme des Gehäuses der Schlauchpumpe eingelegt wird, kann es sich ebenfalls um ein wiederverwendbares Teil oder um ein Teil des Einwegsystems handeln.

Das Hindurchführen der Hülse, mittels derer der Drain in die Wunde eingebracht wird, durch das Gewebe erfolgt mittels einer Nadel, an deren hinterem Ende die Hülse angehängt wird. Um die Hülse sicher mit dieser Nadel verbinden, aber auch einfach wieder von ihr lösen zu können, ist bei einer bevorzugten Ausführungsform am hinteren Ende der Nadel ein in die Hülse einsteckbarer Zapfen vorgesehen, der mittels eines Bajonettverschlusses mit der Hülse verbindbar ist.

Um mit Sicherheit ausschließen zu können, daß das über die Hautoberfläche überstehende Ende der Hülse mit der Hautoberfläche und darauf eventuell befindlichen Erregern in Berührung kommen kann, ist bei einer bevorzugten Ausführungsform auf dem Zapfen der Nadel eine Schutzhülse angeordnet, an die sich eine einstückig mit ihr ausgebildete, verformbare Manschette anschließt, welche den vorderen Endabschnitt der Hülse übergreift und vollständig nach außen hin abdeckt. Diese Schutzhülse und die sich an sie anschließende Manschette werden zusammen mit der Nadel von der Hülse getrennt, so daß deren Endabschnitt erst nach dieser Trennung freigelegt wird. Liegt die Manschette im Bereich ihres freien Endabschnittes dicht an der Hülse an oder ist mit dieser fest verbunden, beispielsweise verklebt, was ebenfalls möglich ist und einen verstärkten Schutz gegen eine Kontaminierung des vorderen Endes der Hülse ergibt, dann braucht vor der Trennung der Nadel von der Hülse nur durch einen Schnitt mit dem Skalpell der festgelegte Endabschnitt der Manschette von dem übrigen Teil der Manschette abgetrennt zu werden.

Eine Nadel mit einer derartigen Manschette läßt sich mit Vorteil auch dann einsetzen, wenn keine Hülse verwendet wird, sondern das vom Schlauch getrennte hintere Ende des Drains von der der Wunde zugekehrten Seite her durch das Gewebe hindurchgeführt wird. Die Manschette sorgt auch hier dafür, daß das mit dem Schlauch zu verbindende Ende des Drains nicht mit auf der Hautoberfläche vorhandenen Erregern in Berührung kommen kann, also steril bleibt.

Um die Hülse trotz einer einfachen und kostengünstigen Ausbildung ohne Schwierigkeiten vom Schlauch entfernen zu können, nachdem sie nach dem Einlegen des Drains aus dem Gewebe herausgezogen worden ist, besteht sie vorzugsweise aus Kunststoff und ist mit einem sich über ihre gesamte Länge erstreckenden Schlitz sowie, diesem diametral gegenüberliegend, einem Filmscharnier versehen. Die beiden Hälften der Hülse brauchen dann nur aufgeklappt zu werden, um vom Schlauch abgenommen werden zu können. Um an der Hülse keine Verschlußelemente vorsehen zu müssen, welche die Hülse im geschlossenen Zustand halten, während sie in das Gewebe eingeführt wird, ist sie bei einer bevorzugten Ausführungsform zumindest auf einem Teil ihrer Länge von einem dünnen Schlauch aus schneidbarem Kunststoff umhüllt. Es braucht dann nur dieser Schlauch durch einen Schnitt mit dem Skalpell in Längsrichtung durchgetrennt zu werden, um die Hülse öffnen und von dem vom Drain zur Pumpe führenden Schlauch abnehmen zu können.

Im folgenden ist die Erfindung anhand von in der Zeichnung dargestellten Ausführungsbeispielen im einzelnen erläutert. Es zeigen:

Fig. 1    eine schematische Darstellung der Absaugvorrichtung,

Fig. 2    eine Frontansicht des Steuergerätes und der Sekret-Absaugpumpe,

Fig. 3    eine perspektivisch dargestellte Ansicht des Steuergerätes und der Sekret-Absaugpumpe,

Fig. 4    eine Draufsicht auf das Steuergerät und die Sekret-Absaugpumpe,

Fig. 5    eine Draufsicht auf den in die Sekret-Absaugpumpe einzulegenden Schlauchabschnitt samt Halter,

Fig. 6    das Schaltbild der Absaugvorrichtung,

Fig. 7    eine schematische Darstellung einer ersten Ausführungsform einer Nadel mit daran angekuppelter Hülse nach dem Hindurchführen letzterer durch das eine Wunde begrenzende Gewebe,

Fig. 8    eine schematische Darstellung der durch das Gewebe hindurchgeführten Hülse und eines Drains zu Beginn des Einführens in die Hülse,

Fig. 9    eine schematische Darstellung der Lage des Drains nach dem Hindurchführen durch die Hülse und nach dem Herausziehen der Hülse aus dem Gewebe,

Fig. 10    einen unvollständig dargestellten Längsschnitt durch die Hülse und die Nadel gemäß der ersten Ausführungsform in vergrößertem Maßstab,

Fig. 11    einen Schnitt nach der Linie XI-XI der Fig. 10,

Fig. 12    einen unvollständig dargestellten Längsschnitt einer Nadel gemäß einer zweiten Ausführungsform in Verbindung mit einer Hülse.

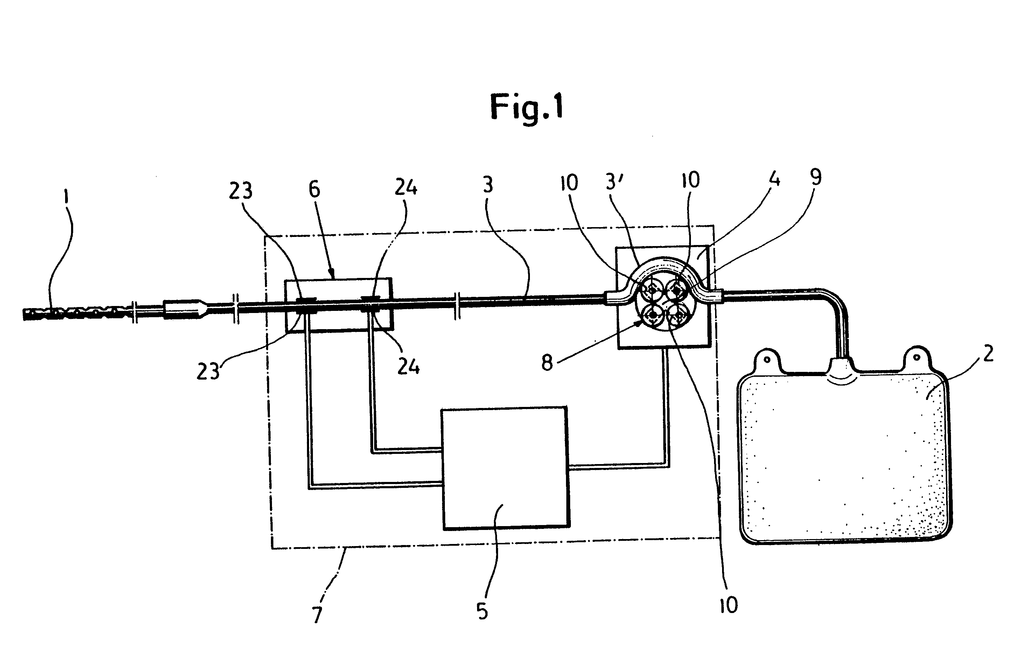

Eine Absaugeinrichtung zum Absaugen von Sekretflüssigkeit aus Wunden besteht, wie Fig. 1 zeigt, aus einem in die Wunde zu legenden Drain 1, einem vom Drain zu einem Sammelbehälter 2 verlaufenden Schlauch 3, einer Schlauchpumpe 4, einer die Pumpe steuernden Steuereinrichtung 5 und einer Sensoreinrichtung 6. Die Steuereinrichtung 5 und die Sensoreinrichtung 6 sind im Ausführungsbeispiel in einem Gehäuse 7 untergebracht, auf das die Schlauchpumpe 4 aufgesetzt ist. Der Sammelbehälter 2 ist vorzugsweise ein Kunststoffbeutel. Auch der Schlauch 3 besteht im Ausführungsbeispiel mit Ausnahme des in die Schlauchpumpe 4 einzulegenden Abschnittes 3', bei dem es sich um einen Silikongummischlauch handelt, aus Kunststoff. Da der Schlauch 3 unlösbar mit dem Sammelbehälter 2 und dem Drain 1 verbunden ist, bilden der Drain 1, der Schlauch 3 und der Sammelbehälter 2 ein geschlossenes System, dessen mit der Wunde und der Wundsekretflüssigkeit in Berührung kommende Teile steril gehalten werden können und das für den Gebrauch und während des Gebrauchs nicht geteilt werden muß.

Die Schlauchpumpe 4 weist einen auf einer drehbar gelagerten Antriebsachse angeordneten Pumpenkopf 8 auf, welcher aus einem fest mit der Antriebsachse verbundenen, im Ausführungsbeispiel sternförmigen Träger 9 und je einer an jedem der vier Arme des Trägers 9 angeordneten Rolle 10 besteht, welche über das Armende überstehen und auf je einer zur Antriebsachse parallelen Achse symmetrisch zu dieser drehbar gelagert sind.

Um den Abschnitt 3' des Schlauches 3, auf den die Rollen 10 zur Erzielung der Pumpwirkung einwirken müssen, bequem in die bezüglich des Pumpenkopf es 8 richtige Position bringen und in dieser Position auch halten zu können, ist ein aus Kunststoff bestehender, rinnenartiger Halter 11 vorgesehen, der an seinen beiden Enden mit je einem den Schlauch erfassenden, federnden Klammerelement 12 versehen ist. Der Halter 11 ist entweder als ein wiederverwendbarer Teil der Schlauchpumpe ausgebildet oder als Teil des Wegwerfsystems, das ständig mit dem Abschnitt 3' verbunden bleibt.

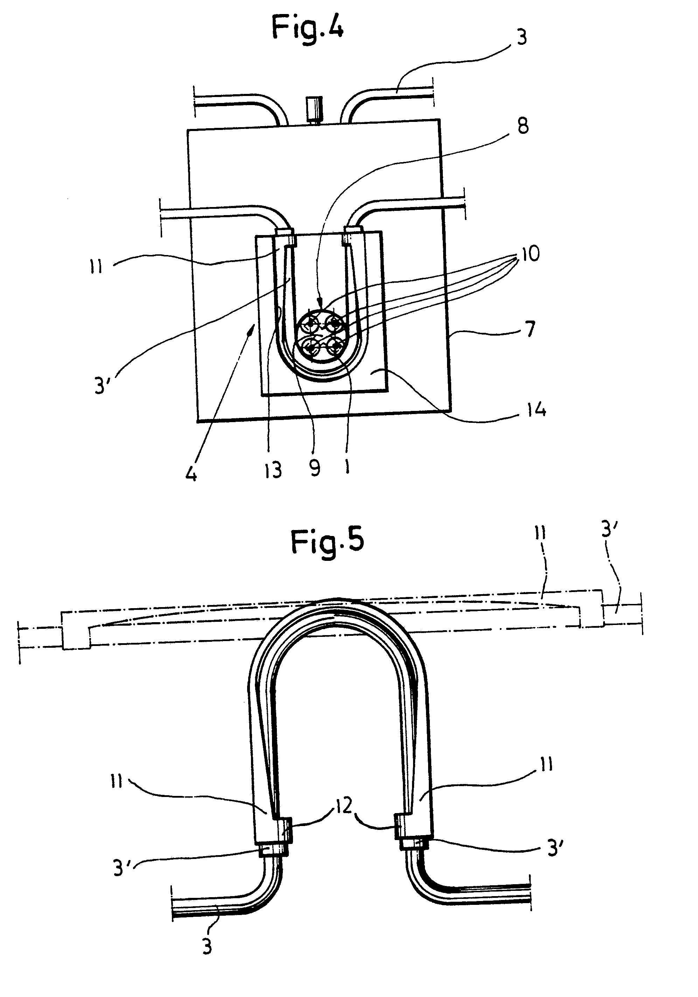

Aus der in Fig. 5 mit gestrichelten Linien dargestellten, gestreckten Lage wird der Halter 11 zusammen mit dem Abschnitt 3' des Schlauches 3 in die in Fig. 5 mit ausgezogenen Linien dargestellte U-Form gebogen und dann in eine Aufnahme 13 eines den Pumpenkopf 8 tragenden Pumpengehäuses 14 eingelegt, wobei die Aufnahme 13 den U-förmig gebogenen Halter 11 auf dessen dem Schlauchabschnitt 3' abgekehrter Seite abstützt. Nicht dargestellte Rastelemente sichern den Halter 11 gegen eine Verschiebung in seiner Längsrichtung.

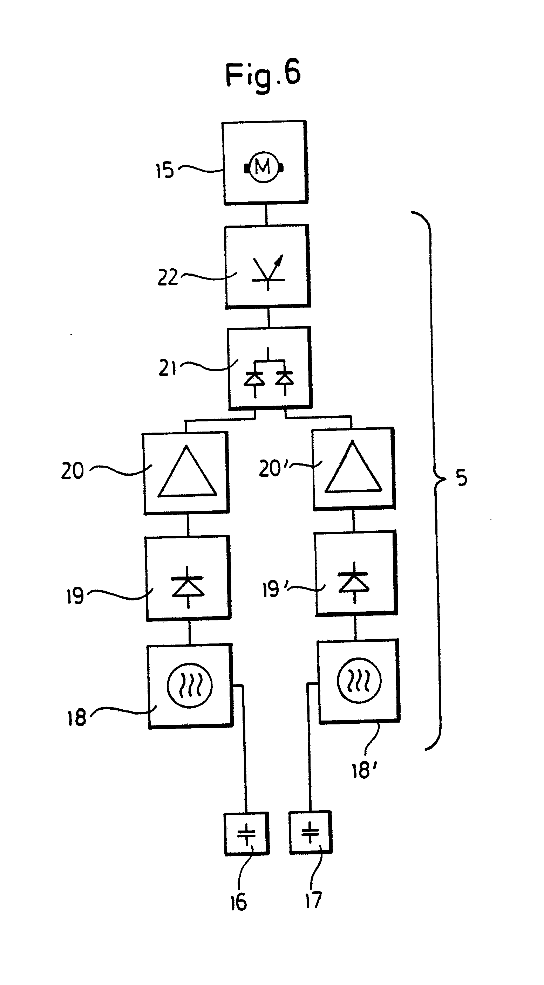

Der Pumpenkopf 8 wird über ein Getriebe von einem in bekannter Weise ausgebildeten und daher in Fig. 6 nur schematisch dargestellten Elektromotor 15 angetrieben, dessen Drehzahl veränderbar ist.

Die Einstellung der Drehzahl des Elektromotors 15 erfolgt mittels der Steuereinrichtung 5, die eine an zwei kapazitive Sekretsensoren 16 und 17 angeschlossene Intervallsteuerung bildet. Wie Fig. 6 zeigt, weist die Steuereinrichtung 5 zwei Oszillatoren 18 bzw. 18' auf, denen je ein Spannungsgleichrichter 19 bzw. 19' nachgeschaltet ist, der die Ausgangsspannung des Oszillators gleichrichtet. Der Ausgang dieser beiden Spannungsgleichrichter ist an den Eingang je eines Verstärkers 20 bzw. 20' angeschlossen. Sowohl diese beiden Verstärker als auch die beiden Spannungsgleichrichter und die beiden Oszillatoren sind gleich ausgebildet. Der Ausgang des Verstärkers 20 ist an den einen Eingang, der Ausgang des Verstärkers 20' an den anderen Eingang einer logischen Schaltung 21 angeschlossen, in welcher eine ODER-Verknüpfung folgt. Die Ausgangsspannung der logischen Schaltung 21 wird dem Eingang eines Leistungsverstärkers 22 zugeführt, der in der Energieversorgungsleitung des Elektromotors 15 der Schlauchpumpe 4 liegt.

Im Schwingkreis des einen Oszillators 18 liegt der erste kapazitive Sekretsensor 16, im Schwingkreis des Oszillators 18' der zweite kapazitive Sekretsensor 17. Beide Sekretsensoren weisen zwei Elektroden 23 bzw. 24 auf, zwischen denen zwei in Abstand Schlauchlängsrichtung und im Abstand voneinander liegende Schlauchabschnitte zu liegen kommen. Dieser Abstand der beiden Elektrodenpaare voneinander ist größer als die übliche Länge von Luftblasen, welche in der Sekretflüssigkeit mitgeführt werden.

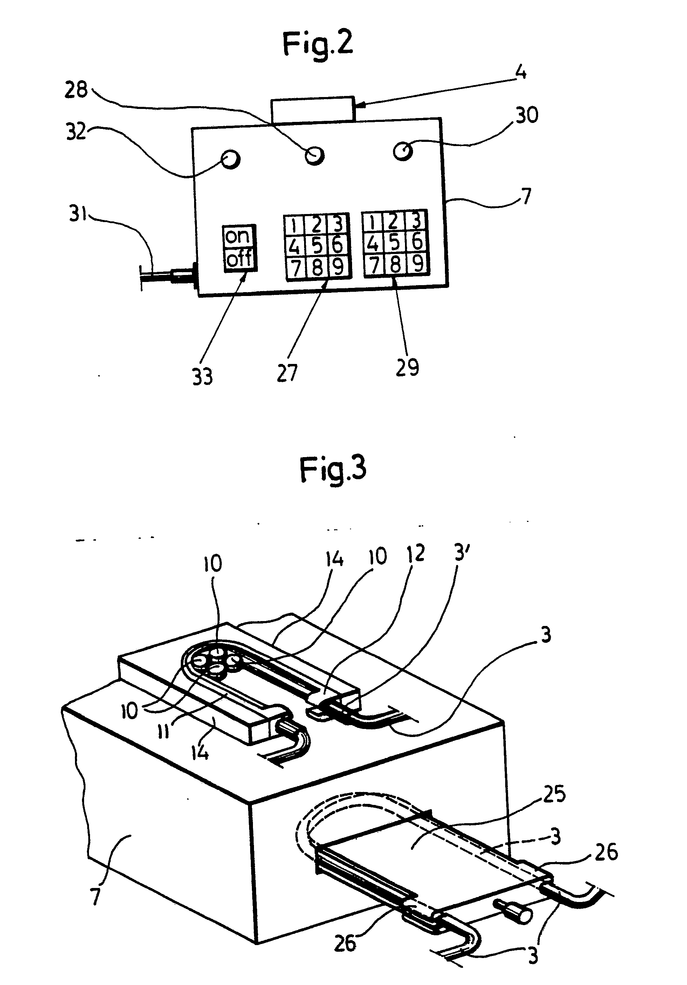

Da die beiden Elektrodenpaare 23 und 24 im Inneren des Gehäuses 7 angeordnet sind, ist ein aus Kunststoff bestehender Meßkammer-Einschub 25 vorgesehen, der, wie Fig. 3 zeigt, eine U-förmige Außenkontur sowie eine an den Durchmesser des Schlauches 3 angepaßte Dicke hat. Die die Außenkontur begrenzende Seitenfläche des Meßkammer-Einschubes 25 bildet eine Rinne zur Aufnahme eines zwischen dem Drain 1 und dem Abschnitt 3' liegenden Abschnittes des Schlauches 3. Je ein angeformtes Klammerelement 26 hält den Schlauch 3 in Anlage am Meßkammer-Einschub 25, der durch eine Öffnung in der Rückwand des Gehäuses 7 hindurch in die im Inneren des Gehäuses 7 vorgesehene Meßkammer eingeführt werden kann. In der Meßkammer sind die beiden Elektrodenpaare 23 und 24 so angeordnet, daß

zwischen ihnen der eine bzw. der andere Schenkel des durch die Anlage am Einschub 25 U-förmig gekrümmten Schlauches 3 liegen. Da der Meßkammer-Einschub 25 ein billiges Kunststoffteil ist, kann er nach Beendigung der Absaugung aus dem Gehäuse 7 herausgezogen und zusammen mit dem Schlauch 3 weggeworfen werden. Selbstverständlich wäre es aber auch möglich, den Schlauch 3 vom Einschub 25 zu lösen und letzteren wieder zu verwenden.

Solange keine Sekretflüssigkeit oder nur eine sehr geringe Sekretmenge anfällt, sich zwischen den Elektroden 23 und 24 also nur der Schlauch 3 und Luft oder allenfalls eine geringe Sekretmenge befindet, halten die beiden Sekretsensoren 16 und 17 die Oszillatoren 18 bzw. 18' in einem Zustand, in dem ihre Ausgangsspannung einen niedrigen Pegel hat. Die beiden Ausgangsspannungen, die in der logischen Schaltung 21 miteinander verknüpft werden, haben wegen ihres niedrigen Pegels zur Folge, daß der Leistungsverstärker 22 nur eine so geringe Spannung abgibt, daß der an ihn angeschlossene Elektromotor 15 mit einer wählbaren Drehzahl läuft, die einer gewünschten minimalen Saugleistung entspricht. An der Frontplatte des Gehäuses 7 ist eine Folientastatur 27 zur Einstellung unterschiedlicher minimaler Drehzahlen vorgesehen. Statt einer solchen Tastatur könnte aber auch beispielsweise ein Drehknopf verwendet werden. Solange der Elektromotor 15 mit seiner niedrigen Drehzahl läuft leuchtet eine oberhalb der Folientastatur 27 vorgesehene Kontrolleuchte 28 auf. Außerdem ist jeweils diejenige Taste der Folientastatur 27 beleuchtet, die zur Drehzahleinstellung betätigt worden ist.

Sobald einer der beiden Sekretsensoren 16 oder 17 eine größere Menge Sekretflüssigkeit erkennt, weil der Sekretanfall angestiegen ist, erreicht die Ausgangsspannung eines der beiden Oszillatoren 18 oder 18' einen so hohen Pegel, daß die Ausgangsspannung der logischen Schaltung 21 eine Ausgangsspannung des Leistungsverstärkers 22 bewirkt, welche zu einer erhöhten Drehzahl des Elektromotors 15 und damit zu einer erhöhten Leistung der Schlauchpumpe 14 führt. Die erhöhte Drehzahl des Elektromotors 15 ist mittels einer zweiten Folientastatur 29 wählbar, die ebenfalls auf der Frontplatte des Gehäuses 7 vorgesehen ist. Auch statt dieser Tastatur könnten selbstverständlich andere Einstellmittel, beispielsweise ein Einstellknopf, verwendet werden. Eine zweite Kontrolleuchte 30 in der Frontplatte leuchtet, während der Elektromotor 15 mit der erhöhten Drehzahl läuft. Die erhöhte Leistung der Schlauchpumpe 4 wird so gewählt, daß sie ausreicht, um die maximal anfallende Sekretmenge in den Sammelbehälter 2 abführen zu können.

Luftblasen, die in der Sekretflüssigkeit mitgeführt werden, führen zwar dazu, daß zunächst der erste Sekretsensor 16 und etwas später der zweite Sekretsensor 17 einen niedrigen Pegel der Ausgangsspannung des zugehörigen Oszillators 18 bzw. 18' bewirken. Da jedoch dann, wenn der zweite Sekretsensor eine Luftblase erkennt, letztere den Wirkungsbereich des ersten Sekretsensors bereits verlassen hat und deshalb der Pegel der Ausgangsspannung des vom ersten Sekretsensor gesteuerten Oszillators bereits wieder hoch ist, läuft der Elektromotor 15 mit der höheren Drehzahl weiter. Eine Umschaltung der Schlauchpumpe auf die minimale Leistung wird von den beiden Sekretsensoren 16 und 17 erst dann ausgelöst, wenn beide nur noch Luft oder eine geringe Sekretmenge erkennen. In diesem Betriebs zustand bleibt die Schlauchpumpe 4, bis wieder eine eine Umschaltung auf die höhere Leistung erforderlich machende Sekretmenge von wenigstens einem der beiden Sekretsensoren 16 und 17 erkannt wird.

Normalerweise wird die Energie für den Antrieb des Elektromotors 15 und für den Betrieb der Steuereinrichtung 5 über eine Netzanschlußleitung 31 aus dem Netz bezogen. Um bei einem Netzausfall einen Stillstand des Elektromotors 15 zu verhindern, enthält das Gehäuse 7 aber auch eine Notstrombatterie, welche bei einem Stromausfall die Energieversorgung übernimmt. Bei Notstrombetrieb leuchtet eine dritte, in der Frontplatte des Gehäuses 7 vorgesehene Kontrolleuchte 32 auf. Ferner ist in der Frontplatte noch ein Netzschalter 33 vorgesehen.

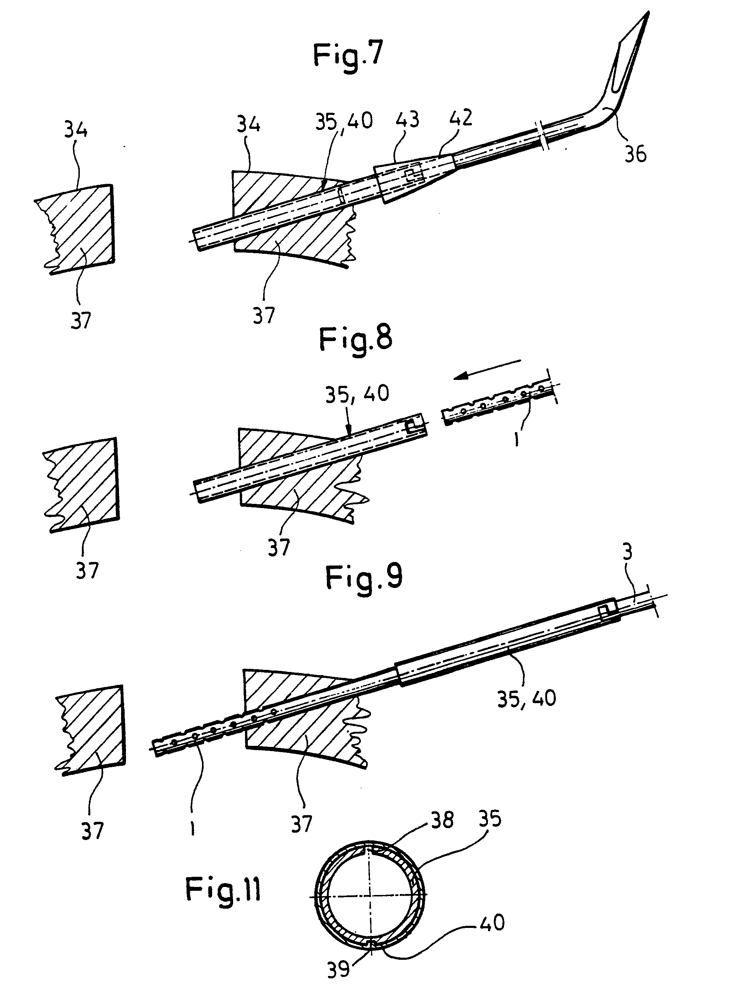

Um zu verhindern, daß beim Einführen des Drains 1 in die Wunde Erreger, welche sich auf der Hautoberfläche 34 befinden, in die Wunde gelangen können, wird eine sterile, aus Kunststoff bestehende Hülse 35 verwendet, deren Innendurchmesser etwas größer ist als der Außendurchmesser des Drains 1. Diese Hülse 35 wird, wie Fig. 7 zeigt, mit Hilfe einer aus Metall bestehenden Nadel 36 von innen her in das die Wunde begrenzende Gewebe 37 eingeführt und so weit durch dieses Gewebe 37 hindurchgeführt, bis die Hülse 35 ein Stück weit aus der Hautoberfläche 34 herausragt.

Die Hülse 35 ist, wie Fig. 11 zeigt, mit einem sich über ihre gesamte Länge erstreckenden Längsschlitz 38 versehen. Diametral zum Längsschlitz 38 liegt eine ein Filmscharnier bildende, sich ebenfalls über die gesamte Länge der Hülse erstreckende Einschnürung 39. Ein dünner, über die Hülse 35 gezogener Folienschlauch 40 aus einer gut schneidbaren Kunststoffolie hält den Längsschlitz 38 geschlossen

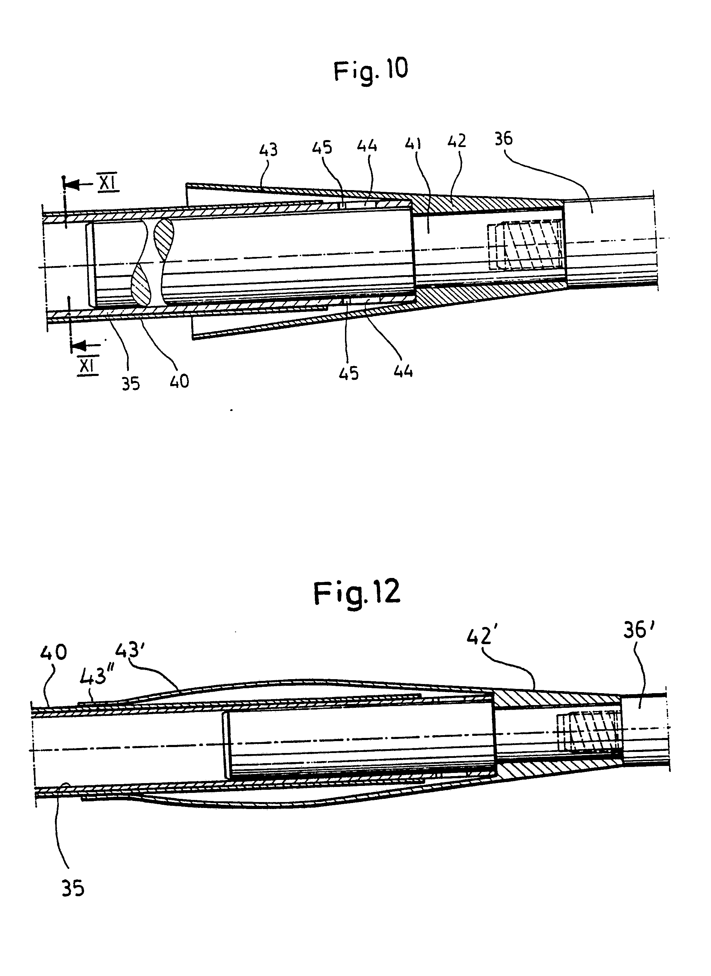

Wie Fig. 10 zeigt, ist an das hintere Ende der Nadel 36 ein abgesetzter Zapfen 41 gleichachsig angeschraubt. Auf dessen sich an das Nadelende anschließenden, im Durchmesser kleineren Abschnitt ist eine aus Kunststoff bestehende Konusbuchse 42 aufgeschoben, deren Außendurchmesser an dem an die Nadel 36 angrenzenden Ende gleich dem Außendurchmesser des zylindrischen Spießes ist und sich bis zu seinem anderen Ende hin gleichmässig auf einen Wert vergrößert, der etwas größer ist als der Außendurchmesser der Hülse 35. An dieses Ende schließt sich einstückig eine Manschette 43 an, deren Wandstärke so gering gewählt ist, daß die Manschette 43 sich leicht deformieren läßt. Die Manschette 43 hat, wie Fig. 10 zeigt, eine konische Form, so daß sie eine Verlängerung der konischen Buchse 42 bildet, welche den an die konische Buchse 42 angrenzenden Endabschnitt der Hülse 35 sowie den zugehörigen Endabschnitt des Folienschlauches 40 ein Stück weit übergreift. Der im Durchmesser größere Abschnitt des Zapfens 41 ist in seinem Außendurchmesser an den Innendurchmesser der Hülse 35 angepaßt, so daß diese über diesen Abschnitt des Zapfens 41 bis zur Anlage am Ende der konischen Buchse 42 geschoben werden kann. Zwei diametral angeordnete, radiale Vorsprünge 44 am Zapfen 41 in dem die Hülse 35 aufnehmenden Teil bilden zusammen mit je einem abgewinkelten Schlitz 45 an dem an die Konusbuchse 42 anliegenden Endabschnitt einen Bajonettverschluß, mittels dessen die Hülse 35 über den Zapfen 41 formschlüssig mit der Nadel 36 verbunden und durch eine Drehung leicht von der Nadel 36 getrennt werden kann. Nachdem die Hülse 35 mit Hilfe der Nadel 36 so weit von innen her durch das Gewebe 37 hindurchgeführt worden ist, daß die Manschette 43 auf ihrer gesamten Länge freiliegt, wird die Nadel 36 im Sinne des Lösens des Bajonettverschlusses relativ zur Hülse 35 gedreht und dann zusammen mit dem Zapfen 41, der Konusbuchse 42 und der Manschette 43 vom Hülsenende abgezogen. Das aus der Hautoberfläche 34 herausragende Ende der Hülse 35 ist dank der Manschette 43 auf einer so großen Länge nicht mit der Hautoberfläche 34 in Berührung gekommen, daß bei dem nun erfolgenden Einführen des Drains 1 in das nach außen weisende Ende der Hülse 35 der Drain mit Sicherheit nicht mit Teilen der Hülse 35 in Berührung kommt, die kontaminiert sind, d.h., in Berührung mit der Hautoberfläche 34 gekommen sind.

Nachdem der Drain 1 in die Wunde eingelegt worden ist, wird die Hülse 35 aus dem Gewebe 37 heraus über den Drain oder den Schlauch 3 gezogen, wie dies Fig. 9 zeigt. Nun braucht nur noch mit dem Skalpell der Folienschlauch 40 in Längsrichtung durchgeschnitten zu werden, um die Hülse 35 aufklappen und vom Schlauch 3 abnehmen zu können. Nach dem Verschließen der Wunde wird dann zunächst die Schlauchpumpe mit der geringen Saugleistung in Gang gesetzt. Sie erhöht dann selbsttätig dank der Sekretsensoren 16 und 17 im Bedarfsfalle die Saugleistung.

In Fig. 12 ist eine modifizierte Ausführungsform einer Nadel dargestellt, welche sich von der vorstehend beschriebenen und insbesondere in Fig. 10 dargestellten Nadel nur dadurch unterscheidet, daß die Manschette 43' der auf das hintere Nadelende aufgesetzten Konusbuchse 42' im Bereich ihres der Konusbuchse abgekehrten Endabschnittes 43'' dicht an der Außenmantelfläche des Folienschlauches 40 anliegt und mit diesem verklebt ist. Hierdurch ist mit Sicherheit ausgeschlossen, daß Erreger in den von der Manschette 43' übergriffenen Abschnitt der Hülse 35 und des Folienschlauches 40 gelangen können. Wegen weiterer Einzelheiten über die Ausgestaltung der Nadel 36', der Konusbuchse 42' und der Verbindung der Nadel 36' mit der Hülse 35 wird auf Fig. 10 und die zugehörigen Erläuterungen verwiesen, da insoweit keine Unterschiede bestehen.

## Patentansprüche

1. Vorrichtung zum Absaugen von Sekretflüssigkeit aus einer Wunde mit einem Drain (1), der über einen Schlauch (3) mit einem Sekretsammelbehälter (2) dicht verbunden ist, und einer Pumpe, die mit dem Sekretsammelbehälter (2), dem Schlauch (3) und dem Drain (1) zu einem sterilen System vereinigt ist, dadurch gekennzeichnet, daß die Pumpe eine an den Schlauch (3) ansetzbare Schlauchpumpe (4) ist, die an eine Intervallsteuerung (5) angeschlossen ist, der wenigstens ein Sekretsensor (16, 17) zugeordnet ist, welcher während der Erkennung von aus dem Drain (1) abfließender Sekretflüssigkeit die Intervallsteuerung (5), die zuvor und danach einen Pumpbetrieb mit einer Mindestleistung bewirkt, in einem eine erhöhte Leistung ergebenden Zustand hält.

2. Vorrichtung nach Anspruch 1, dadurch gekennzeichnet, daß der Sekretsensor (16, 17) an dem zwischen dem Drain (1) und der Schlauchpumpe (4) liegenden Abschnitt des Schlauches (3) angeordnet ist.

3. Vorrichtung nach Anspruch 2, dadurch gekennzeichnet, daß der Sekretsensor (16, 17) als ein durch die Wandung des Schlauches (3) von der Sekretflüssigkeit getrennter, kapazitiver oder induktiver Geber ausgebildet ist.

4. Vorrichtung nach Anspruch 3, dadurch gekennzeichnet, daß der Sekretsensor (16, 17) zwei den Schlauch zwischen sich aufnehmende Elektroden (23, 24) aufweist.

5. Vorrichtung nach einem der Ansprüche 1 bis 4, dadurch gekennzeichnet, daß an dem Abschnitt des Schlauches (3) zwischen der Schlauchpumpe (4) und dem Drain (1) ein zweiter Sekretsensor (17) in Strömungsrichtung gegenüber dem ersten Sekretsensor (16) versetzt angeordnet ist, der beim Auftreten von im Sekret mitgeführter Luftblasen eine Umschaltung der Schlauchpumpe (4) auf die minimale Leistung durch den ersten Sekretsensor (16) verhindert.

6. Vorrichtung nach Anspruch 5, dadurch gekennzeichnet, daß die durch die beiden Sekretsensoren (16, 17) zur Auslösung einer Umschaltung der Schlauchpumpe (4) erzeugten Signale durch eine ODER-Schaltung (21) miteinander verknüpft sind.

7. Vorrichtung nach einem der Ansprüchen 1 bis 6, dadurch gekennzeichnet, daß jeder vorhandene Sekretsensor (16, 17) im Schwingkreis eines Oszillators (18, 18') liegt, dessen Ausgangsspannung hinsichtlich ihres Pegels vom Sekretsensor (16, 17) abhängt.

8. Vorrichtung nach einem der Ansprüchen 1 bis 7, dadurch gekennzeichnet, daß die Intervallsteuerung (5) Stellelemente (27, 29) für die veränderbare Einstellung der Größe der minimalen und der maximalen Saugleistung der Schlauchpumpe (4) hat.

9. Vorrichtung nach einem der Ansprüche 1 bis 9, dadurch gekennzeichnet, daß die Schlauchpumpe (4) außen an einem die Intervallsteuerung (5) enthaltenden Gehäuse (7) angeordnet ist.

10. Vorrichtung nach einem der Ansprüche 1 bis 9, gekennzeichnet durch einen/das Gehäuse (7) der Intervallsteuerung (5) einführbaren Einschub (25), der mit einer Aufnahme für einen zwischen der Schlauchpumpe (4) und dem Drain (1) liegenden Abschnitt des Schlauches (3) versehen ist und im eingeführten Zustand diesen Schlauchabschnitt in der für eine Abtastung durch den Sekretsensor (16) oder die Sekretsensoren (16, 17) erforderlichen Lage hält.

11. Vorrichtung nach einem der Ansprüche 1 bis 10 in Kombination mit einer Nadel (36, 36') für das Verlegen des Drains (1) der Vorrichtung in die Wunde, dadurch gekennzeichnet, daß die Nadel (36, 36'), zum Hindurchführen eines mit der Nadel lösbar verbundenen, hohlzylindrischen Körpers (35, 40) durch das der Wunde benachbarte Gewebe (37) von dessen der Wunde zugekehrten Seite gegen die das Gewebe nach außen hin begrenzende Körperoberfläche (34), am hinteren Ende eine aus einem Kunststoff bestehende, sterile Schutzhülse (42; 42') aufweist, an die sich eine einstückig mit ihr ausgebildete, elastisch verformbare Manschette (43, 43') anschließt, deren Innendurchmesser größer ist als der Außendurchmesser des hohlzylindrischen Körpers (35, 40) und die dessen vorderen, mit der Nadel (36, 36') lösbar verbundenen Endabschnitt aufnimmt.

12. Vorrichtung nach Anspruch 11, dadurch gekennzeichnet, daß im Bereich des freien Endes der Manschette (43') ein ringzonenförmiger Abschnitt (43'') dicht am hohlzylindrischen Körper (35, 40) anliegt.

13. Vorrichtung nach Anspruch 11 oder 12, dadurch gekennzeichnet, daß der hinterer Endabschnitt der Nagel (36, 36') als ein in den vorderen Endabschnitt des hohlzylindrischen Körpers (35, 40) eingreifender Zapfen (41) ausgebildet ist, der mittels eines Bajonettverschlusses (44, 45) lösbar mit dem hohlzylindrischen Körper (35, 40) verbunden ist.

14. Vorrichtung nach einem der Ansprüche 11 bis 13, dadurch gekennzeichnet, daß der hohlzylindrische Körper eine Hülse (35) ist, die aus Kunststoff besteht sowie mit einem sich über ihre gesamte Länge erstreckenden und ihre Wandung durchtrennenden Schlitz (38) und diametral zu diesem Schlitz mit einem sich ebenfalls über die gesamte Hülsenlänge erstreckenden Filmscharnier (39) versehen ist.

15. Vorrichtung nach Anspruch 14, dadurch gekennzeichnet, daß die Hülse (35) zumindest auf einem Teil ihrer Länge von einem dünnen Schlauch (40) aus einem mittels eines Messers durchschneidbaren Kunststoff umhüllt ist.

## Claims

1. Device for extracting by vacuum secretion from a wound, comprising a drain (1) which is tightly connected by a tube (3) to a secretion collector (2), and with a pump combined with the secretion collector (2), the tube (3) and the drain (1) to constitute one sterile system, characterised in that the pump is a tube pump (4) which can be applied to the tube (3) and which is connected to an interval control system (5) with which at least one secretion sensor (16, 17) is associated which, during recognition of secretion flowing from the drain (1), maintains in a condition which provides enhanced output the interval control system (5) which previously and subsequently causes the pump to operate on minimum power.

2. Device according to Claim 1, characterised in that the secretion sensor (16, 17) is disposed on that portion of the tube (3) which is located between the drain (1) and the tube pump (4).

3. Device according to Claim 2, characterised in that the secretion sensor (16, 17) is constructed as a capacitative or inductive transmitter separated from the secreted fluid by the wall of the tube (3).

4. Device according to Claim 3, characterised in that the secretion sensor (16, 17) has two electrodes (23, 24) which hold the tube between them.

5. Device according to one of Claims 1 to 4, characterised in that there is disposed on the portion of the tube (3) between the tube pump (4) and the drain (1) a second secretion sensor (17) which in the direction of flow is offset to the first secretion sensor (16) which, in the event of air bubbles being entrained in the secretion, prevents the tube pump (4) being switched over to minimal power by the first scretion sensor (16).

6. Device according to Claim 5, characterised in that the signals generated by the two secretion sensors (16, 17) to trigger switch over of the tube pump (4) are linked together by an OR circuit (21).

7. Device according to one of Claims 1 to 6, characterised in that each existing secretion sensor (16, 17) lies in the oscillating circuit of an oscillator (18, 18'), the output voltage from which depends for its level upon the secretion sensor (16, 17).

8. Device according to one of Claims 1 to 7, characterised in that the interval control system (5) has positioning elements (27, 29) for the variable setting of the magnitude of minimum and maximum suction performance of the tube pump (4).

9. Device according to one of Claims 1 to 8, characterised in that the tube pump (4) is disposed on the outside of a housing (7) containing the interval control system (5).

10. Device according to one of Claims 1 to 9, characterised by, adapted for insertion into the housing (7) of the interval control system (5), a push-in assembly (25) provided with a housing for a portion of the tube (3) located between the tube pump (4) and the drain (1) and which, in the inserted condition, maintains this portion of tube in the location needed for scanning by the secretion sensor (16) or the secretion sensors (16, 17).

11. Device according to one of Claims 1 to 10 in conjunction with a needle (36, 36') for laying the drain (1) of the apparatus into the wound, characterised in that the needle (36, 36'), for passing a hollow cylindrical body (35, 40) separately connected to the needle through the tissue (37) adjacent the wound from the side which is towards the wound towards the surface of the body (34) which outwardly bounds the tissue, comprises at the rear end a sterile protective sleeve (42, 42') consisting of a synthetic plastics material and adjacent to and integral with which there is an elastically deformable cuff (43, 43'), the inside diameter of which is greater than the outside diameter of the hollow cylindrical body (35, 40), and which accommodates the front end portion of the latter, which is separably connected to the needle (36, 36').

12. Device according to Claim 11, characterised in that there is in the region of the free end of the cuff (43') an annular portion (43") which bears tightly against the hollow cylindrical body (35, 40).

13. Device according to Claim 11 or 12, characterised in that the rear end portion of the needle (36, 36') is constructed as a peg (41) which engages into the front end portion of the hollow cylindrical body (35, 40) and which is separably connected to the hollow cylindrical body (35, 40) by means of a bayonet coupling (44, 45).

14. Device according to one of Claims 11 to 13, characterised in that the hollow cylindrical body is a sleeve (35) which consists of synthetic plastics material and which has extending over its entire length and separating its walls, a slit (38), a film hinge (39) being provided which extends diametrically to this slit and likewise over the entire length of the sleeve.

15. Device according to Claim 14, characterised in that at least over a portion of its length, the sleeve (35) is encased in a thin tube (40) of a synthetic plastics material which can be cut through by means of a knife.

## Revendications

1. Dispositif destiné à aspirer les sécrétions liquides d'une plaie, comprenant un drain (1) qui est relié à joint étanche à un récipient collecteur de sécrétions (2) par l'intermédiaire d'un tuyau (3), et une pompe qui est réunie au récipient collecteur de sécrétions (2), au tuyau (3) et au drain (1) en formant un système stérile, caractérisé en ce que la pompe (4) est une pompe tubulaire qui peut se monter sur le tuyau (3), et qui est raccordée à une commande d'intervalle (5) associée à au moins un capteur de sécrétions (16, 17) qui, lorsqu'il détecte une sécrétion liquide qui sort du drain (1), maintient la commande d'intervalle (5) dans un état assurant une puissance élevée, tandis que, avant et après, cette commande fait fonctionner la pompe à une puissance minimale.

2. Dispositif selon la revendication 1, caractérisé en ce que le capteur de sécrétions (16, 17) est agencé sur le sent du tuyau (3) qui est situé entre le drain (1) et la pompe tubulaire (4).

3. Dispositif selon la revendication 2, caractérisé en ce que le capteur de sécrétions (16, 17) est constitué par un capteur capacitif ou inductif qui est isolé des sécrétions liquides par la paroi du tuyau (3).

4. Dispositif selon la revendication 3, caractérisé en ce que le capteur de sécrétions (2) comprend deux électrodes (23, 24) qui reçoivent le tuyau entre elles.

5. Dispositif selon l'une des revendications 1 à 4, caractérisé en ce que, sur le segment du tuyau (3) compris entre la pompe tubulaire (4) et le drain (1), est agencé un deuxième capteur de sécrétions (17), qui est décalé dans le sens de l'écoulement par rapport au premier capteur de sécrétions (16), et qui, lors de l'apparition de bulles d'air entraînées dans la sécrétion, évite que la pompe tubulaire (4) ne soit commutée sur la puissance minimale par le premier capteur de sécrétions (16).

6. Dispositif selon la revendication 5, caractérisé en ce que les signaux produits par les deux capteurs de sécrétions (16, 17) pour déclencher la commutation de la pompe tubulaire (4) sont combinés entre eux par un circuit OU (21).

7. Dispositif selon l'une des revendications 1 à 6, caractérisé en ce que chaque capteur de sécrétions (16, 17) présent dans le système est intercalé dans le circuit oscillant d'un oscillateur (18, 18') dont le niveau de la tension de sortie est déterminé par le capteur de sécrétions (16, 17).

8. Dispositif selon une des revendications 1 à 7, caractérisé en ce que la commande d'intervalle (5) est équipée d'éléments de réglage (27, 29) permettant de régler la puissance d'aspiration minimale et la puissance d'aspiration maximale de la pompe tubulaire (4) sur des valeurs prédéterminées.

9. Dispositif selon une des revendications 1 à 8, caractérisé en ce que la pompe tubulaire est montée extérieurement sur un boîtier (7) qui renferme la commande d'intervalle (5).

10. Dispositif selon une des revendications 1 à 9, caractérisé par un tiroir (25) pouvant être engagé dans le boîtier (7) de la commande d'intervalle (5), qui est muni d'un logement pouvant recevoir un segment du tuyau (3) compris entre la pompe tubulaire (4) et le drain (1), et qui, dans l'état engagé, maintient ce segment de tuyau dans la position nécessaire pour pouvoir être palpé par le capteur de sécrétions (16) ou par les capteurs de sécrétions (16, 17).

11. Dispositif selon une des revendications 1 à 10, en combinaison avec une aiguille (36, 36') destinée à mettre le drain (1) du dispositif en place dans la plaie, caractérisé en ce que, pour enfiler un corps creux cylindrique (35, 40) fixé à l'aiguille par une liaison démontable, à travers les tissus (37) adjacents à la plaie, à partir du côté de ces tissus qui est dirigé vers la plaie, en direction de la surface (34) de la peau qui limite les tissus à l'extérieur, l'aiguille (36, 36') présente en son extrémité arrière une gaine protectrice stérile (42, 42') réalisée en une matière plastique, à laquelle se raccorde une manchette élastiquement déformable (43, 43') qui est formée d'une seule pièce avec cette gaine, dont le diamètre intérieur est supérieur au diamètre extérieur du corps cylindrique creux (35, 40) et qui reçoit le segment d'extrémité avant de ce corps, qui est assemblé à l'aiguille (36, 36') par une liaison démontable.

12. Dispositif selon la revendication 11, caractérisé en ce que, dans la région de l'extrémité libre de la manchette (43'), un segment (43'') présentant la forme d'une zone annulaire est appuyé à joint étanche sur le corps creux cylindrique (35, 40).

13. Dispositif selon la revendication 11 ou 12, caractérisé en ce que le segment d'extrémité arrière de l'aiguille (36, 36') est constitué par un embout (41) qui s'engage dans le segment d'extrémité avant du corps creux cylindrique (35, 40), et qui est relié au corps cylindrique creux (35, 40) par une liaison démontable, au moyen d'un assemblage à baîonnette (44, 45).

14. Dispositif selon l'une des revendications 11 à 13, caractérisé en ce que le corps cylindrique creux est une gaine (35), qui est composée de matière plastique et est munie d'une fente (38) s'étendant sur toute sa longueur et traversant sa paroi, ainsi que, dans une position diamétrale par rapport à cette fente, d'une charnière-film (39) qui s'étend elle aussi sur toute la longueur de la gaine.

15. Dispositif selon la revendication 14, caractérisé en ce que la gaine (35) est entourée, au moins sur une partie de sa longueur, par un tuyau mince (40) fait d'une matière plastique que l'on peut couper au moyen d'un couteau.

# Fig.1

0 128 388

# Fig.2

# Fig.3

## Fig.4

## Fig.5

# Fig.6

15

22

21

20    20'

5

19    19'

18    18'

16    17

# Fig.7

34

34    35,40    43    42    36

37

37    37

# Fig.8

35, 40

37

37    1

37

# Fig.9

3

35, 40

37

37    1

# Fig.11

38    35

39    40

# Fig. 10

# Fig.12